**Europäisches Patentamt**

**European Patent Office**

**Office européen des brevets**

(19)

(11) Veröffentlichungsnummer: **0 085 724**
**B1**

(12)

# EUROPÄISCHE PATENTSCHRIFT

(45) Veröffentlichungstag der Patentschrift:
**07.01.87**

(21) Anmeldenummer: **82100828.1**

(22) Anmeldetag: **05.02.82**

(51) Int. Cl.⁴: **A 61 M 5/00,** A 61 M 5/31

(54) **Diabetiker-Set.**

(43) Veröffentlichungstag der Anmeldung:
**17.08.83 Patentblatt 83/33**

(45) Bekanntmachung des Hinweises auf die Patenterteilung:
**07.01.87 Patentblatt 87/2**

(84) Benannte Vertragsstaaten:
**AT BE CH DE FR GB IT LI LU NL SE**

(56) Entgegenhaltungen:
**EP - A - 0 076 872**
**FR - A - 952 088**
**FR - A - 975 595**
**US - A - 1 625 035**
**US - A - 1 634 029**
**US - A - 1 910 033**
**US - A - 2 740 516**

(73) Patentinhaber: **Petz Electro, Friesenstrasse 791,
CH-3185 Schmitten (CH)**

(72) Erfinder: **Petz, Günter, Flachslander Strasse 8,
D-8500 Nürnberg (DE)**

(74) Vertreter: **Göbel, Matthias, Dipl.-Ing., Pruppacher
Hauptstrasse 5-7, D-8501 Pyrbaum-Pruppach (DE)**

ACTORUM AG

## Beschreibung

Die Erfindung betrifft einen Diabetiker-Set mit einem durch einen kappenartigen Deckel verschliessbaren Aufnahmeteil für Insulinflaschen und Injektionsspritzen, wobei der Aufnahmeteil durch einen etuiförmigen Hohlkörper und einem die Insulinflaschen und Injektionsspritzen aufnehmenden kastenförmigen Einsatz gebildet ist, der die Insulinflaschen und Injektionsspritzen von aussen zugänglich haltert, der Hohlkörper auf der dem Deckel abgewandten Seite eine verschliessbare Kammer aufweist und der Deckel verschieb- und verschwenkbar am Einsatz gelagert ist. Ein solches Diabetiker-Set ist in dem unter Artikel 54(3) EPÜ fallenden Dokument EP-A-0 076 872 beschrieben.

Bei einem bekannten Diabetiker-Set (US-A 1 625 035) nimmt ein einseitig offener und durch einen Klappdeckel verschliessbarer Kasten eine Insulinflasche und eine Injektionsspritze auf. Der Klappdeckel und der Kasten sind bei diesem Diabetiker-Set durch ein Scharniergelenk miteinander verbunden, was komplizierte Ausgestaltungen für Klappdeckel und Kasten ergibt. Bei dem Diabetiker-Set gemäss dem Dokument EP-A-0 076 872 ist der Aufnahmeteil durch einen am Einsatz verschieb- und verschwenkbar gelagerten Deckel verschliessbar. Der Deckel ist hierzu aufwendig mittels laschenförmigen Ansätzen in Nuten des Einsatzes geführt, wodurch sich komplizierte Ausbildungen für den Einsatz und Deckel einstellen. Ausserdem sind die Klappbewegungen für den Deckel nur umständlich durchzuführen.

Es ist Aufgabe der Erfindung bei einem Diabetiker-Set der eingangs genannten Art, den baulichen Aufwand zu verringern und die Handhabung zu erleichtern.

Erfindungsgemäss ist hierzu vorgesehen, dass der Einsatz und der Deckel durch eine mit einer Teillänge im Einsatz oder Deckel fest angeordneten und mit einer weiteren Teillänge im Deckel bzw. Einsatz verschieblich geführten Welle miteinander verbunden sind und dass der Deckel in der Offenstellung des Aufnahmeteils mittels der Welle schwenkbar am Einsatz gehalten ist. Bevorzugt ist der Einsatz axial in den Hohlkörper einschiebbar und in der inneren Endstellung desselben auf hohlkörperfeste Leisten abstützbar und zur Festlegung im Hohlkörper durch Eingreifen eines am Einsatz angebogenen Federstreifens in eine Ausnehmung des Hohlkörpers verrastet gehalten. Hierdurch ist erreicht den Deckel durch einen Verschiebevorgang zunächst aus der Schliessstellung in die Offenstellung zu bringen und durch einen nachfolgenden Schwenkvorgang, z.B. durch Aufdrücken des Daumens, aus dem Bereich der Insulinflaschen und Injektionsspritzen fortzubewegen, sodass für den Benutzer die Möglichkeit gegeben ist, vermittels einer Einhandbedienung des Diabetiker-Sets unbehindert die Injektionsspritzen zu entnehmen und Insulin aufzusaugen. Hierbei gibt die den Deckel haltende Welle die Voraussetzung, dass sich das vielfach unhygienische Ablegen des Deckels erübrigt. Zum Verschliessen des Diabetiker-Sets bedarf es einer einfachen Rückschwenkung des Deckels und eines Aufschiebevorgangs desselben auf den Einsatz. Die Aufteilung des Aufnahmeteils in einen Hohlkörper und einen Einsatz erlaubt auch eine wirtschaftlich günstige Montage des Aufnahmeteils durch einen Steckvorgang des Einsatzes und dessen selbsttätige Festlegung mittels der Rastenglieder. Schliesslich entspricht es der Erfindung, entweder die Welle im Deckel fest und im Einsatz verschieblich zu führen oder in kinematischer Umkehrung die Welle im Einsatz festzulegen und den Deckel verschiebbar auf der Welle anzuordnen.

In Ausgestaltung des Diabetiker-Sets ist als Einsatz ein im wesentlichen prismatischer Hohlkörper aus aneinanderfügbaren Körperhälften vorgesehen. Die Körperhälften können miteinander durch angespritzte Gelenke, insbesondere zwischen diesen ausgeformte Dünnschichtstreifen verbunden sein. Durch Anschwenken der Körperhälften aineinander ist der Einsatz aufrichtbar, wobei in der angeklappten Stellung die Körperhälften durch Rasten aneinander gehalten sind. Von Vorteil hat sich erwiesen, wenn der Einsatz getrennte Aufnahmeräume für die Insulinflaschen und die Injektionsspritzen aufweist. Dies lässt sich einfach dadurch erreichen, dass die Körperhälften mit Kammerabschnitten versehen sind, die sich beim Anschwenken der Körperhälften aneinander zu unabhängigen Aufnahmekammern für die Injektionsspritzen ergänzen.

Es ist weiter vorgesehen, die im Hohlkörper auf der dem Deckel abgewandten Seite angeordnete Kammer stirnseitig offen und durch einen auf dem Hohlkörper aufschiebbaren Deckel verschliessbar zu machen. Um die Handhabung dieses Deckels zu vereinfachen, kann der Deckel auf einer im Hohlkörper oder Einsatz verschieblich gelagerten deckelfesten Welle mit dem Hohlkörper bzw. Einsatz schwenkbar verbunden sein. Hierdurch verbleibt der Deckel mit dem Aufnahmeteil verbunden, wodurch das bereits erwähnte unhygienische Ablegen von Teilen des Diabetiker-Sets vermieden ist. Es versteht sich, dass die Kammer als Aufnahmeraum für beliebiges Zubehör und/oder Tablettenbehälter dienen kann.

Die Erfindung ist anhand eines in der Zeichnung dargestellten Ausführungsbeispiels erläutert. Hierin zeigen:

Figur 1 einen Diabetiker-Set geschlossen in Vorderansicht,

Figur 2 einen Diabetiker-Set im Schnitt,

Figur 3 einen Diabetiker-Set geschlossen in Seitenansicht,

Figur 4 einen Schnitt nach der Linie IV–IV der Figur 2,

Figur 5 einen Einsatz in Draufsicht,

Figur 6 einen durch Körperhälften gebildeten Einsatz in Draufsicht,

Figur 7 einen Einsatz aufgeklappt in Aussenansicht,

Figur 8 einen Einsatz aufgeklappt in Innenansicht und

Figur 9 einen Teilschnitt nach der Linie IX–IX der Figur 7, vergrössert.

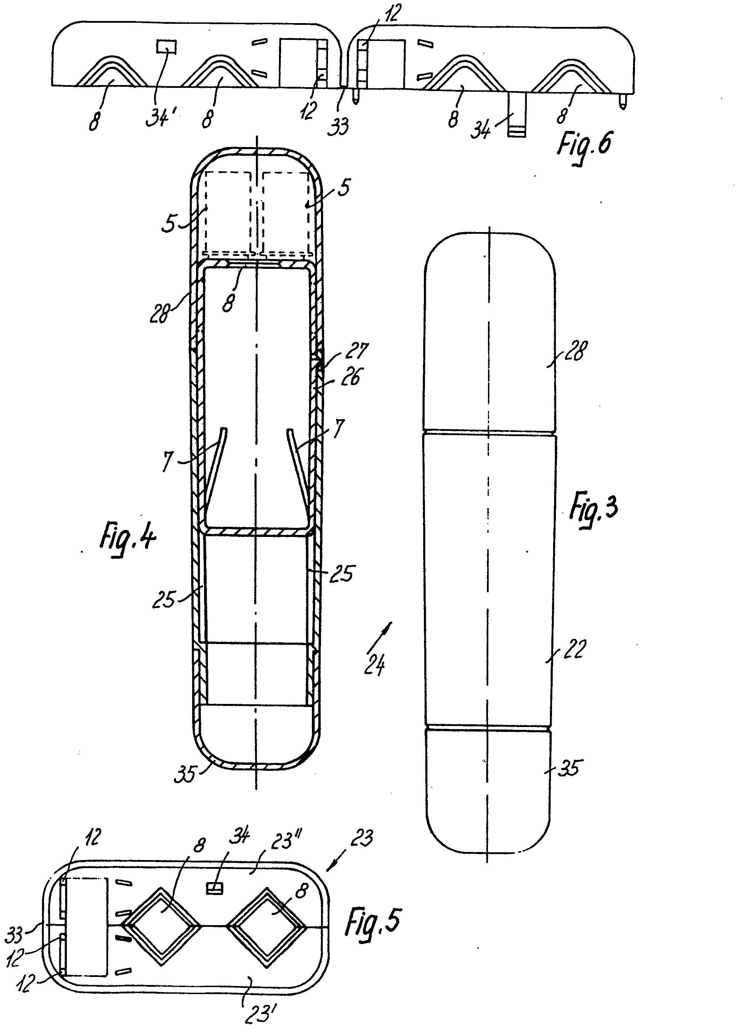

Der Diabetiker-Set weist einen durch einen Hohlkörper 22 und einen kastenförmigen Einsatz 23 gebildeten etuiförmigen Aufnahmeteil 24 für zwei Insulinflaschen 4 und Injektionsspritzen 5 auf. Der Einsatz 23 ist in den Hohlkörper 22 bis zur Anlage an hohlkörperfeste Leisten 25 eingeschoben und durch einen am Einsatz 23 angeformten und ausgebogenen Federstreifen 26, der in eine Ausnehmung 27 des Hohlkörpers 22 einrastet (Fig. 4) mit dem Hohlkörper 22 fest verbunden. Der Aufnahmeteil 24 ist durch einen auf den Einsatz 23 aufschiebbaren Deckel 28 verschliessbar, der erfindungsgemäss durch eine Welle 29 mit dem Einsatz 23 verbunden ist. Die Welle 29 ist im Deckel 28 fest angeordnet und in eine Verdickung 30 des Einsatzes 23 verschieblich geführt, wodurch, durch einen möglichen Schwenkvorgang in der Offenstellung des Deckels 28, eine freie Handhabung der Injektionsspritzen und der Insulinflaschen 4 gewährleistet ist. Die Injektionsspritzen 5 sind im Einsatz 23 mittels federnd elastischer Anformungen 12 und die Insulinflaschen 4 durch Haltelappen 7 fixiert und zusätzlich die Insulinflaschen 4 mit den Flaschenhälsen in Durchstecköffnungen 8 des Einsatzes 23 abgestützt. Die Durchstecköffnungen 8 sind in ihrer Querschnittsgrösse so bemessen, dass ein Herausfallen der Insulinflaschen 4 aus dem Einsatz sicher unterbleibt. Oberhalb der Haltelappen 7 sind als Durchblicköffnungen dienende Ausnehmungen 11 vorgesehen, die ein Erkennen der Insulinflaschen und deren Inhalte erlauben. Zu einer sicheren Unterbringung der Injektionsspritzen 5 ragen diese in im Einsatz 23 ausgebildete Aufnahmekammern 31 ein.

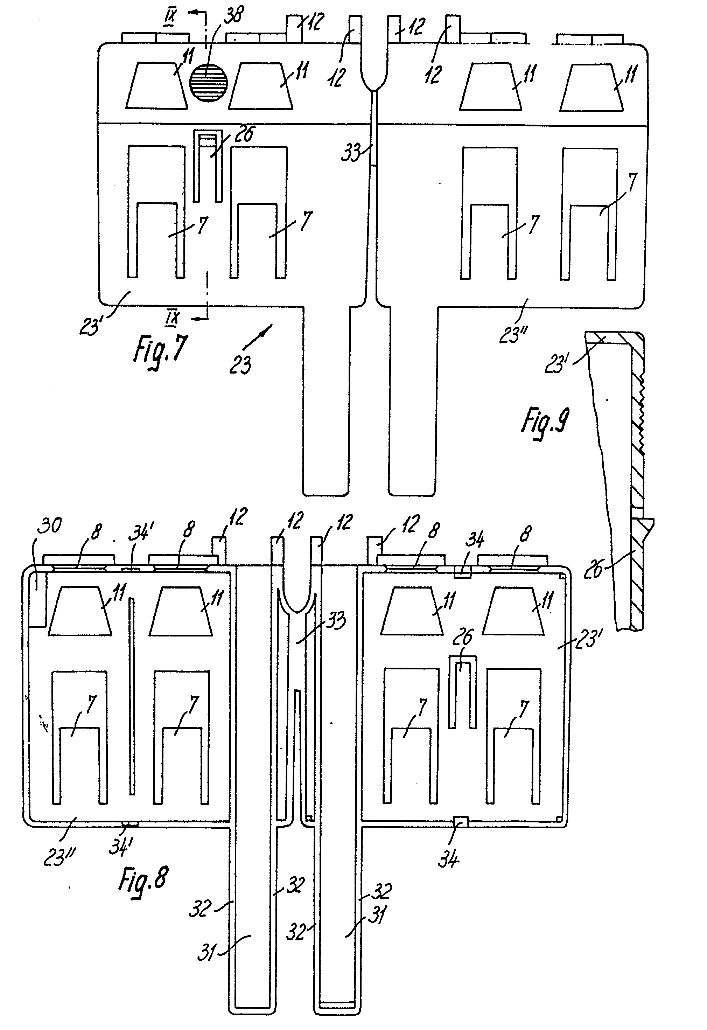

Wie die Figuren 6 bis 8 zeigen, ist der Einsatz 23 durch einen Körperhälften 23' und 23'' aufweisenden Formteil gebildet, die zur Bildung der Aufnahmekammern 31 für die Injektionsspritzen mit vorbereiteten angeformten Trennwänden 32 versehen sind und sich beim Aneinanderklappen der Körperhälften 23', 23'' (Fig. 6) zu Aufnahmekammern 31 ergänzen. Die beiden Körperhälften 23', 23'' sind durch einen Dünnschichtstreifen 33 klappbar miteinander verbunden und mittels hakenförmiger Rastenstreifen 34, die in Öffnungen 34' der anderen Körperhälfte eingreifen, aneinander festlegbar. An seinem unteren Ende ist der Aufnahmeteil 24 unter Bildung einer Kammer 37 stirnseitig offen und durch einen aufschiebbaren Deckel 35 verschliessbar. Der Deckel 35 greift hierbei in eine Falz 36 des Hohlkörpers 22 ein. Abweichend kann der Deckel 35 zusätzlich durch eine Welle (nicht gezeigt) mit dem Hohlkörper 22 oder dem Einsatz 23 verbunden sein, wodurch eine Verschiebebewegung mit nachfolgender Abschwenkung desselben erfolgen kann.

Durch Abschwenken des Deckels 35 ist die Kammer 37 über die Stirnseite frei, wodurch eine sichere und einfache Entnahme von in der Aufnahmekammer 37 untergebrachtem Zubehör, z.B. Alkoholtücher oder Tablettenbehälter möglich ist. Durch die Anordnung einer Grifffläche 38 an der Körperhälfte 23' ist durch Aufdrücken ein leichtes Schliessen bzw. Öffnen des Einsatzes 23 möglich.

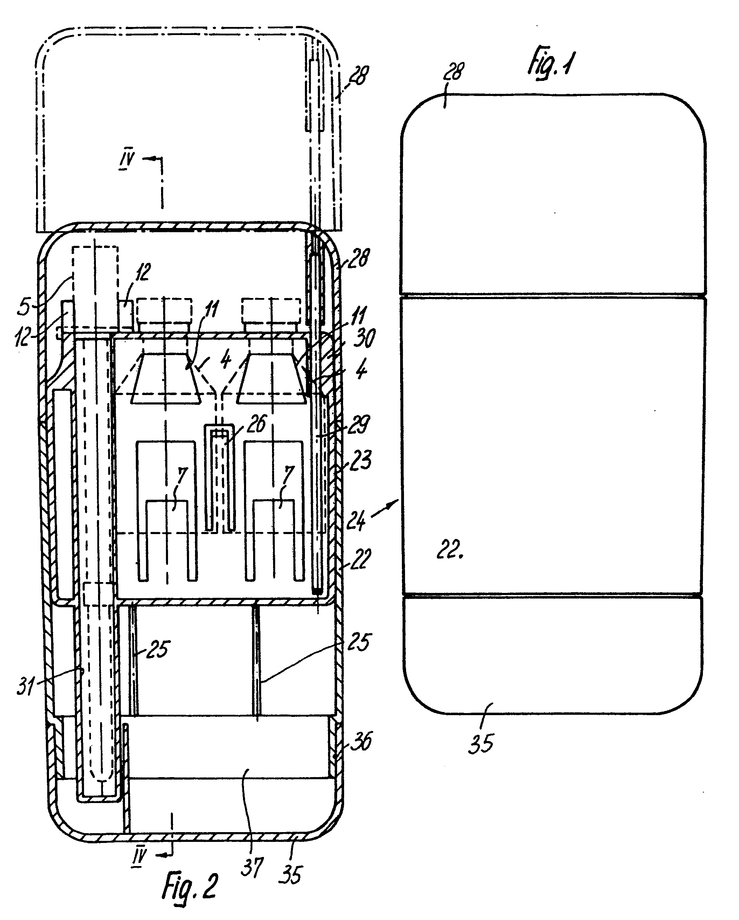

Zur Benutzung ist der Diabetiker-Set mit seinem Aufnahmeteil 24 durch eine Hand des Benutzers zu erfassen. Mit der anderen Hand kann der Benutzer den Deckel 28 nach oben vom Einsatz 23 abschieben und durch nachfolgendes Schwenken um die Welle 29 in die Offenstellung bringen. Nach seiner Freigabe verbleibt der Deckel 28 am Einsatz 23. Mit seiner nunmehr freien Hand kann der Benutzer eine Injektionsspritze 5 aus den Anformungen 12 und den Kammern 31 herausnehmen und zur Durchführung des Insulinspritzenvorganges Insulin aus den Insulinflaschen 4 aufziehen. Nach dem Spritzvorgang ist die Injektionsspritze 5 in die Kammer 31 zurückzustecken, wobei sie von den Anformungen 12 festgelegt wird. Durch Zurückschwenken des Deckels 28 und Aufschieben desselben auf den Einsatz 23 ist der Aufnahmeteil 24 des Diabetiker-Sets verschliessbar (Fig. 1, 2).

## Patentansprüche

1. Diabetiker-Set mit einem durch einen kappenartigen Deckel (28) verschliessbaren Aufnahmeteil (24) für Insulinflaschen (4) und Injektionsspritzen (5), wobei der Aufnahmeteil durch einen etuiförmigen Hohlkörper (22) und einem die Insulinflaschen und Injektionsspritzen aufnehmenden kastenförmigen Einsatz (23) gebildet ist, der die Insulinflaschen und Injektionsspritzen von aussen zugänglich haltert, der Hohlkörper auf der dem Deckel (28) abgewandten Seite eine verschliessbare Kammer (37) aufweist und der Deckel (28) verschieb- und verschwenkbar am Einsatz (23) gelagert ist, wobei der Einsatz (23) und der Deckel (28) durch eine mit einer Teillänge im Einsatz (23) oder Deckel (28) fest angeordneten und mit einer weiteren Teillänge im Deckel (28) bzw. Einsatz (23) verschieblich geführten Welle (29) miteinander verbunden sind und der Deckel (28) in der Offenstellung mittels der Welle (29) schwenkbar am Einsatz (23) gehalten ist.

2. Diabetiker-Set nach Patentanspruch 1, dadurch gekennzeichnet, dass der Einsatz (23) axial in den Hohlkörper (22) einschiebbar und in der inneren Endstellung auf hohlkörperfeste Leisten (25) stützbar ist und dass durch Eingreifen eines am Einsatz (23) angebogenen Federstreifens (26) in eine Ausnehmung (27) des Hohlkörpers (22) der Einsatz (23) am Hohlkörper (22) verrastet festgelegt ist.

3. Diabetiker-Set nach Patentanspruch 1, dadurch gekennzeichnet, dass der Einsatz (23) durch einen im wesentlichen prismatischen Hohlkörper aus aneinanderfügbaren Körperhälften (23', 23'') gebildet ist.

4. Diabetiker-Set nach Patentanspruch 3, dadurch gekennzeichnet, dass die Körperhälften (23', 23'') durch Gelenke schwenkbar miteinander verbunden sind.

5. Diabetiker-Set nach Patentanspruch 4, dadurch gekennzeichnet, dass als Gelenke zwischen den Körperhälften (23', 23'') ausgeformte Dünnschichtstreifen (33) dienen.

6. Diabetiker-Set nach Patentanspruch 3, dadurch gekennzeichnet, dass die Körperhälften (23', 23'') zu Aufnahmekammern (31) für die Injektionsspritzen (5) sich ergänzende Kammerabschnitte (32) aufweisen.

7. Diabetiker-Set nach Patentanspruch 1, dadurch gekennzeichnet, dass die Kammer (37) an der dem Deckel (28) abgewandten Stirnseite offen ausgebildet ist und durch einen auf den Hohlkörper (22) aufschiebbaren kappenartigen Deckel (35) verschliessbar ist.

8. Diabetiker-Set nach Patentanspruch 7, dadurch gekennzeichnet, dass der Deckel (35) auf den Hohlkörper (22) aufschiebbar und durch eine am Hohlkörper oder Einsatz (23) verschieblich gelagerten deckelfesten Welle (29) mit dem Hohlkörper bzw. Einsatz schwenkbar verbunden ist.

## Claims

1. Diabetic set with holder section (24) closable with a cap type cover (28) for insulin bottles (4) and hypodermics (5), whereby the holder section consists of a case-shaped hollow part (22) and a boxshaped insert (23) for accommodating the insulin bottles and hypodermics which accommodates the insulin bottles and hypodermics accessibly from the outside, the hollow part on the side facing away from the cover (28) has a closable chamber (37) and the cover (28) is supported movable and tiltable on the insert (23), whereby the insert (23) and the cover (28) are connected together by a permanently fixed section located in the insert (23) or cover (28) and by a further section in the cover (28) and insert (23) in the form of a movable guided shaft (29) and the cover (28) in the open position is held movable on the insert (23) by means of the shaft (29).

2. Diabetic set in accordance with patent claim 1, characterised by the insert (23) being pushed axially into the hollow part (22) and in the inner final position is supported on strips (25) fixed to the hollow part and that by the engagement of a closed spring strip (26) in a recess on the insert (27) of the hollow part (22) the insert (23) is firmly engaged on the hollow part (22).

3. Diabetic set in accordance with patent claim 1, characterised by the insert (23) consisting of an almost prismatic hollow part of two sections (23', 23'') which can be joined together.

4. Diabetic set in accordance with patent claim 3, characterised by two sections (23', 23'') being connected with hinges for tilting.

5. Diabetic set in accordance with patent claim 4, characterised by shaped thin-film strips serving as hinges between the two sections (23', 23'').

6. Diabetic set in accordance with patent claim 3, characterised by two sections (23', 23'') serving as complementary chamber sections (32) to the accommodating chambers (31) for the hypodermics (5).

7. Diabetic set in accordance with patent claim 1, characterised by the chamber (37) facing away from the front side of the cover (28) being open and being closable by a slide-on cap type cover (35) located on the hollow part (22).

8. Diabetic set in accordance with patent claim 7, characterised by the cover (35) being slipped on to the hollow part (22) and through a movable supported shaft (29) fixed to the cover located on the hollow part or insert (23) being hinged to the hollow part and insert.

## Revendications

1. Coffret case-tout pour diabétiques, doté de la partie médiane, une case (24), laquelle est destinée à recevoir les flacons d'insuline (4) et les seringues à injection (5), et qui se ferme par un couvercle en forme de casquette (28). Cette case est constituée d'un corps creux (22) en forme d'étui, et d'une pièce insérée (23) en forme de caissette dans laquelle sont logés les flacons d'insuline et les seringues à injection, et qui maintient ces flacons d'insuline et ces seringues á injection en place de manière à permettre l'accès de l'extérieur. Le corps creux, quant à lui, présente sur le côté opposé au couvercle (28) une chambre (37) qui se ferme, le couvercle (28) lui-même étant logé sur la pièce insérée (23) de manière à pouvoir être déplacé, et à pivoter autour de son articulation. La pièce insérée (23) et le couvercle (28) sont reliés l'un à l'autre à travers un arbre (29) fermement disposé sur une partie de sa longueur dans la pièce insérée (23) ou le couvercle (28), et disposé sur une autre partie de sa longueur dans le couvercle (28) ou dans la pièce insérée (23) de manière à pouvoir coulisser, ledit arbre (29) permettant au couvercle (28) en position d'ouverture de pivoter autour de l'axe verticale de la pièce insérée (23).

2. Coffret case-tout pour diabétiques selon revendication 1, caractérisé en ce que la pièce insérée (23) peut être introduite en sens axial dans le corps creux (22) et s'appuyer en position interne définitive sur des baguettes (25) fixées sur le corps creux, et en ce qu'un ruban élastique (26) replié sur la pièce insérée (23) et venant s'accrocher dans une cavité (27) du corps creux (22), permet à la pièce insérée (23) de s'emboîter fermement dans le corps creux (22).

3. Coffret case-tout pour diabétiques selon revendication 1, caractérisé en ce que la pièce insérée (23) est constituée d'un corps creux de forme essentiellement prismatique, lequel corps étant composé de demicorps (23', 23'') pouvant être alignés les uns aux autres.

4. Coffret case-tout pour diabétiques selon revendication 3, caractérisé en ce que les demicorps (23', 23'') sont reliés les uns aux autres à travers des articulations leur permettant de pivoter.

5. Coffret case-tout pour diabétiques selon revendication 4, caractérisé en ce que la fonction des articulations entre les demi-corps (23', 23'') est assumée par des lamelles façonnées (33).

6. Coffret case-tout pour diabétiques selon revendication 3, caractérisé en ce que les demi-corps (23', 23'') donnant sur les chambres (31) destinées à recevoir les seringues à injection (5), présentent des sections de chambres (32) qui se complètent les unes les autres.

7. Coffret case-tout pour diabétiques selon revendication 1, caractérisé en ce que la chambre (37) située sur le côté frontal opposé au couvercle (28), est conçue à priori en forme non fermée, et peut être fermée avec un couvercle (35) en forme de casquette qui se glisse sur le corps creux (22) en s'encastrant.

8. Coffret case-tout pour diabétiques selon revendication 1, caractérisé en ce que le couvercle (35) peut obturer le corps creux (22) en s'encastrant par glissement, et qu'un arbre (29) lié au couvercle à travers un logement permettant audit arbre de se déplacer le long du corps creux ou de la pièce insérée (23), est assemblé avec le corps creux ou la pièce insérée de manière à pouvoir pivoter autour de son axe.

Fig. 1

Fig. 2

Fig.6

Fig.4

Fig.3

Fig.5

Fig.7

Fig.8

Fig.9